(19) Europäisches Patentamt
European Patent Office
Office européen des brevets

(11) **EP 2 803 385 B1**

(12) **FASCICULE DE BREVET EUROPEEN**

(45) Date de publication et mention de la délivrance du brevet:
**06.04.2016 Bulletin 2016/14**

(51) Int Cl.:
**A61N 1/362** (2006.01)   **A61N 1/368** (2006.01)
**A61N 1/37** (2006.01)   **A61N 1/365** (2006.01)

(21) Numéro de dépôt: **14165460.8**

(22) Date de dépôt: **22.04.2014**

(54) **Resynchroniseur cardiaque implantable à stimulation biventriculaire et détection des pertes de capture et des stimulations anodiques**

Implantierbares CRT-Gerät zur Stimulation beider Ventrikel und zur Erkennung von Erfassungsverlusten und anodischen Stimulationen

Implantable heart re-timer with biventricular stimulation and detection of losses of capture and anode stimulations

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.05.2013 FR 1354330**

(43) Date de publication de la demande:
**19.11.2014 Bulletin 2014/47**

(73) Titulaire: **Sorin CRM SAS**
**92140 Clamart Cedex (FR)**

(72) Inventeurs:
• **Renesto, Fabrizio**
**10013 Borgofranco d'Ivrea (TO) (IT)**
• **Ziglio, Filippo**
**Martignano (TN) 38040 (IT)**

(74) Mandataire: **Dupuis-Latour, Dominique**
**Bardehle Pagenberg**
**10, boulevard Haussmann**
**75009 Paris (FR)**

(56) Documents cités:
EP-A1- 2 495 013   EP-A2- 1 249 254
US-A1- 2010 262 204   US-B1- 6 687 545

**Description**

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" *(Cardiac Resynchronization Therapy)* ou "BVP" (*BiVentricular Pacing).*

Un stimulateur CRT est par exemple divulgué dans le EP 1 108 446 A1 (ELA Medical), qui décrit un dispositif permettant d'appliquer entre les instants respectifs de stimulation des ventricules gauche et droit un délai dit "délai interventriculaire" (DVV ou VVD) variable, ajusté de manière à resynchroniser la contraction des ventricules avec optimisation fine de l'état hémodynamique du patient.

En effet, une stimulation simultanée des deux ventricules n'est pas toujours optimale, car elle n'aboutit pas forcément à une contraction synchrone des deux ventricules du fait, d'une part, des délais de conduction au sein du myocarde qui ne sont pas les mêmes à droite et à gauche et peuvent dépendre de multiples facteurs et, d'autre part, de l'emplacement de la sonde ventriculaire gauche, selon que celle-ci est une sonde enfilée dans le sinus coronaire ou une sonde épicardique. Il est donc souhaitable d'établir un délai entre les deux stimulations, et d'ajuster ce délai pour resynchroniser la contraction des ventricules et assurer ainsi une optimisation fine de l'hémodynamique. Le DVV pourra être nul, positif (le ventricule gauche étant stimulé après le ventricule droit), ou négatif (le ventricule droit étant stimulé après le ventricule gauche).

Les dispositifs CRT incluent en outre un mode de fonctionnement "double chambre" classique dans lequel le dispositif surveille l'activité ventriculaire après un évènement auriculaire spontané (détection d'une onde P de dépolarisation auriculaire) ou stimulé (application d'une impulsion A de stimulation auriculaire). En même temps, le dispositif commence à compter un délai dit "délai atrioventriculaire" ou "délai auriculo-ventriculaire" (DAV ou AVD) tel que si aucune activité spontanée ventriculaire (onde R) n'a été détectée à l'issue de ce délai, alors le dispositif déclenche une stimulation de ce ventricule (application d'une impulsion V).

Des études cliniques ont permis d'observer des résultats souvent spectaculaires pour des patients en insuffisance cardiaque non améliorée par les traitements classiques, dès lors que les paramètres de la thérapie CRT sont ajustés de façon précise en fonction du patient et de la nature de son trouble de la contraction myocardique, tel que dilatation des cavités cardiaques, faible fraction d'éjection, allongement excessif de la durée du complexe QRS (que ce trouble soit spontané ou induit par une stimulation traditionnelle).

Par ailleurs, diverses études cliniques ont montré que l'accélération endocardiaque (ci-après désignée "EA") est un paramètre qui reflète très précisément et en temps réel les phénomènes liés aux mouvements de la cavité cardiaque, et permet de ce fait de fournir des informations très complètes sur la mécanique cardiaque, aussi bien pour un fonctionnement normal que pour un fonctionnement déficient. L'accélération endocardiaque est par exemple mesurée par un accéléromètre intégré dans une sonde endocavitaire, comme décrit par exemple dans le EP 0 515 319 A1 (Sorin Biomedica Cardio SpA).

Dans ce contexte, le EP 1 736 203 A1 (ELA Medical) décrit une technique permettant d'évaluer de façon simple, rapide, automatisée et précise l'incidence des différents paramètres de la stimulation CRT, notamment des délais DAV et DVV, en exécutant un balayage du DAV dans une configuration de stimulation donnée (c'est-à-dire avec un DVV donné), pour tracer une caractéristique donnant la valeur du pic d'accélération endocardiaque PEA (*Peak Endocardial Acceleration*) en fonction du DAV, de manière à sélectionner le couple {DAV, DVV} optimal.

Il est ainsi possible de déterminer, au moment de l'implantation ou ultérieurement, la configuration de stimulation optimale qui optimise l'état hémodynamique du patient.

Cela étant, une thérapie CRT efficace implique en tout état de cause la délivrance d'impulsions de stimulation adéquates, assurant une capture ventriculaire (c'est-à-dire que l'impulsion appliquée a bien induit une dépolarisation de la cavité) à la fois à droite et à gauche, car il s'agit là d'une condition essentielle de la resynchronisation.

Pour vérifier la présence de cette capture, les dispositifs sont munis de moyens de "test de capture" permettant de déterminer si une stimulation a été efficace ou non, et ce sur l'un et l'autre des deux ventricules. Ces moyens opèrent par recherche et analyse de l' "onde évoquée", c'est-à-dire de l'onde de dépolarisation induite par la stimulation de la cavité concernée.

Le test de capture permet en outre d'évaluer le "seuil d'entrainement", c'est-à-dire le niveau minimal d'énergie de stimulation nécessaire pour provoquer la dépolarisation du ventricule (les seuils d'entrainement pouvant être différents entre le site droit et le site gauche). Il convient en effet d'ajuster l'amplitude et/ou la largeur de l'impulsion de stimulation afin, d'une part, de garantir que toute stimulation provoquera une onde évoquée et que, d'autre part, l'énergie délivrée ne sera pas excessive pour ne pas trop obérer la durée de vie de l'implant du fait d'une consommation inutilement élevée. Le test de capture doit être effectué à intervalles réguliers, et il peut être également effectué de façon permanente, cycle-à-cycle.

Par ailleurs, une tendance récente en matière de stimulation biventriculaire est la multiplication des "vecteurs de stimulation", c'est-à-dire la possibilité d'appliquer la stimulation entre différents couples d'électrodes choisis de manière à optimiser la thérapie appliquée, chacune des sondes droite et gauche étant munie à cet effet de plusieurs électrodes sélectivement commutables.

Typiquement, la stimulation "bipolaire" conventionnelle est opérée entre une électrode d'extrémité (*tip*) et une électrode annulaire (*ring*) disposées à proximité sur la sonde droite, ou de même sur la sonde gauche.

Les dispositifs CRT les plus récents proposent aussi une alternative dite "pseudo-bipolaire", dans laquelle la stimulation du ventricule gauche est opérée entre d'une part l'électrode d'extrémité (*tip*) de la sonde ventriculaire gauche, et d'autre part l'électrode annulaire (*ring*) ou un bobinage de défibrillation (*coil*) de la sonde ventriculaire droite. Cette configuration de stimulation "pseudo-bipolaire" du ventricule gauche est susceptible de procurer des avantages notamment en termes de seuils de stimulation, ou en cas de risques de stimulation du nerf phrénique.

Toutefois, l'utilisation de vecteurs de stimulation multiples et différents entraine des fluctuations des seuils de stimulation associés créant un risque corrélatif accru de perte de capture, ainsi que la possible apparition d'un phénomène dit de "stimulation anodique".

Plus précisément, la "stimulation anodique" se caractérise par une inversion de la cathode et de l'anode lors de la stimulation, avec pour conséquence que la stimulation n'est pas délivrée de la manière normale (c'est-à-dire uniquement à la cathode) et que l'onde de dépolarisation induite par cette stimulation inverse n'est pas celle désirée, avec des effets non attendus qui peuvent en résulter.

Ainsi, dans l'exemple donné plus haut d'une stimulation "pseudo-bipolaire" entre une électrode de la sonde gauche (comme cathode) et une électrode de la sonde droite (comme anode) pour stimuler le ventricule gauche, l'onde évoquée est normalement engendrée à la cathode, c'est-à-dire sur le site ventriculaire gauche.

Mais dans certaines situations l'onde de dépolarisation du ventricule gauche ne se crée pas uniquement à la cathode (donc au niveau du ventricule gauche), mais aussi à l'anode (donc au niveau du ventricule droit). De ce fait, l'impulsion destinée à contracter le ventricule gauche va générer également une dépolarisation à droite (phénomène de "stimulation anodique"). Ceci a pour conséquence que l'onde de dépolarisation correspondant à l'impulsion appliquée à gauche va produire au même moment une dépolarisation dans le ventricule droit, faisant perdre le bénéfice d'une stimulation gauche anticipée pendant la thérapie de resynchronisation.

Le risque d'apparition d'une stimulation anodique est accru par l'utilisation de sondes droites bipolaires dédiées utilisant comme anode une électrode de surface relativement faible : en stimulation pseudo-bipolaire, une telle électrode peut en effet engendrer une densité de courant élevée dans son environnement immédiat, augmentant ainsi le risque de déclenchement d'une dépolarisation inopinée du myocarde.

Si la stimulation anodique n'est pas identifiée et corrigée, ce phénomène peut annihiler les effets bénéfiques d'un DVV programmé, et conduire à une resynchronisation sous-optimale, se caractérisant par exemple par une contraction concomitante des deux ventricules, donc une perte du DVV.

Ce phénomène n'est pas rare chez les patients faisant l'objet d'une stimulation biventriculaire. S'il est détecté, le dispositif pourra prendre des mesures appropriées, par exemple la modification de la configuration de stimulation avec basculement vers un autre vecteur de stimulation n'entrainant pas de stimulation anodique (par exemple un vecteur bipolaire pur, un vecteur unipolaire, ou un autre vecteur interventriculaire par sélection d'autres électrodes des sondes droite et/ou gauche).

Certaines conditions favorisent l'apparition d'un phénomène de stimulation anodique, en particulier :

- des amplitudes de stimulation relativement élevées, mais proches du seuil de stimulation ; et
- une configuration de type "vecteur de stimulation intercavité" ou "pseudo-bipolaire", c'est-à-dire dans laquelle une stimulation est appliquée dans une cavité donnée (par exemple le ventricule gauche) avec l'anode sur une sonde implantée dans la cavité opposée (par exemple une électrode d'une sonde ventriculaire droite).

Ces conditions se présentent typiquement pendant l'exécution d'un test de capture, donc de façon répétée et pendant une phase critique d'évaluation de l'efficacité de la stimulation. En effet, le test de seuil de capture commence avec une énergie de stimulation élevée, induisant un risque élevé. Ce risque s'amenuise ensuite au fur et à mesure de la réduction de l'amplitude de stimulation, mais l'algorithme de test aura été leurré en interprétant - à tort - la disparition de la stimulation anodique (qu'il n'a pas identifiée comme telle) par une perte de capture, conduisant à une classification erronée des cycles successifs du test entre cycles capturants et non-capturants.

Le US 6 687 545 B1 décrit un dispositif permettant d'avérer au cours d'un test de capture une telle situation de stimulation anodique, exclusivement à partir des divers potentiels de dépolarisation détectés - ou non - sur les diverses électrodes auriculaires et ventriculaires des sondes endocavitaires et/ou coronaires déjà implantées chez le patient. La proposition de ce brevet vise en effet à éviter le recours à des sondes additionnelles de mesure de la pression sanguine, du débit sanguin, de l'impédance cardiaque ou du mouvement des parois du coeur, qui seraient source de difficultés et de coûts accrus.

Le but de l'invention est de proposer une nouvelle technique d'analyse de l'efficacité de la stimulation biventriculaire permettant d'identifier de manière beaucoup plus précise et fiable non seulement une (véritable) perte de capture à droite et/ou à gauche, mais encore et surtout l'apparition d'un phénomène de stimulation anodique.

En particulier, si la détection du seuil de stimulation est opérée de manière indirecte, avec une détection dans une cavité différente de celle qui est stimulée (typiquement, avec stimulation du ventricule gauche seul et détection des signaux recueillis dans le ventricule droit), les cycles avec stimulation anodique devront être impérativement exclus de l'analyse du signal pour ce test, car ils ne sont pas représentatifs et ne peuvent donc pas être correctement discriminés en cycles capturants ou non-capturants.

Le but de l'invention est de proposer une technique de vérification automatique de l'efficacité de la stimulation biventriculaire, en utilisant notamment les signaux d'accélération endocardiaque (EA) recueillis pendant les procédures d'ajustement automatique des DAV et DVV périodiquement exécutées. Si l'analyse de ces signaux EA, ou de paramètres dérivés de ces signaux, pour des configurations de stimulation différentes révèlent un défaut d'efficacité de la thérapie CRT, alors le dispositif déclenchera une alerte afin de mettre à jour les seuils de capture et/ou modifier la configuration de stimulation.

Plus précisément, l'invention propose un dispositif médical implantable actif de resynchronisation cardiaque par stimulation biventriculaire comportant, de manière en elle-même connue en particulier d'après le US 6 687 545 B1 précité :

- des moyens de détection d'évènements auriculaires et ventriculaires ;
- des moyens de stimulation des ventricules droit et gauche ;
- des moyens aptes à appliquer aux moyens de stimulation un DAV, compté à partir de la détection d'un évènement auriculaire spontané ou stimulé et à l'issue duquel une stimulation du ventricule droit est appliquée en l'absence d'évènement ventriculaire spontané détecté ;
- des moyens aptes à appliquer aux moyens de stimulation un DVV, entre les instants respectifs de stimulation des ventricules droit et gauche ; et
- des moyens de contrôle des moyens de stimulation, aptes à opérer sélectivement soit en un mode droit où seul le ventricule droit est stimulé, soit en un mode gauche où seul le ventricule gauche est stimulé, soit en un mode biventriculaire où les deux ventricules sont stimulés conjointement avec ou sans application d'un DVV.

De façon caractéristique de l'invention, ce dispositif comprend en outre :

- un capteur apte à délivrer un signal d'accélération endocardiaque, EA ;
- des moyens aptes à extraire du signal EA au moins un paramètre EA caractéristique prédéterminé ; et
- des moyens d'évaluation de la stimulation biventriculaire comprenant :

    · des premiers moyens, aptes à comparer les valeurs du paramètre EA caractéristique obtenues respectivement en mode biventriculaire et en mode gauche et à déterminer si l'écart entre ces valeurs excède un premier seuil prédéterminé ;
    · des deuxièmes moyens, aptes à comparer les valeurs du paramètre EA caractéristique obtenues respectivement en mode biventriculaire et en mode droit et à déterminer si l'écart entre ces valeurs excède un deuxième seuil prédéterminé ;
    · des troisièmes moyens, aptes à déterminer si la variation du paramètre EA caractéristique pour différents DAVs en mode gauche excède un troisième seuil donné ;
    · des quatrièmes moyens, aptes à déterminer si la variation du paramètre EA caractéristique pour différents DAVs en mode droit excède un quatrième seuil donné ;
    · des cinquièmes moyens, aptes à comparer les valeurs du paramètre EA caractéristique obtenues en mode biventriculaire respectivement avec un DVV négatif ou positif et à déterminer si l'écart entre ces valeurs excède un cinquième seuil prédéterminé ; et
    · des sixièmes moyens, aptes à déterminer en réponse aux premiers, deuxièmes, troisièmes, quatrièmes et cinquièmes moyens, la survenue d'une éventuelle perte de capture à gauche ou à droite, ou la présence d'un phénomène de stimulation anodique.

Le dispositif comprend en outre, de préférence : des moyens de test préalable d'une pluralité de configurations de stimulation avec différents DVVs pour une pluralité de DAVs ; et des moyens de sélection préalable du paramètre EA caractéristique prédéterminé parmi plusieurs paramètres EA possibles, en fonction des résultats produits par les moyens de test préalable d'une pluralité de configurations, et/ou des moyens de détermination du premier seuil, en fonction des résultats produits par les moyens de test préalable d'une pluralité de configurations.

Les deuxièmes moyens sont notamment mis en oeuvre si les premiers moyens déterminent que l'écart entre les valeurs du paramètre EA caractéristique obtenues en mode biventriculaire et en mode gauche excède le premier seuil. Les troisièmes moyens sont alors mis en oeuvre si les deuxièmes moyens déterminent que l'écart entre les valeurs du paramètre EA caractéristique obtenues en mode biventriculaire et en mode droit n'excède pas le deuxième seuil, et les sixièmes moyens sont aptes à déterminer la survenue d'une perte de capture à gauche si les troisièmes moyens déterminent que la variation du paramètre EA caractéristique pour différents DAVs en mode gauche n'excède pas le troisième seuil.

Les quatrièmes moyens sont notamment mis en oeuvre si les premiers moyens déterminent que l'écart entre les valeurs du paramètre EA caractéristique obtenues en mode biventriculaire et en mode gauche n'excède pas le premier seuil. Les sixièmes moyens sont alors aptes à déterminer la survenue d'une perte de capture à droite si les quatrièmes moyens déterminent que la variation du paramètre EA caractéristique pour différents DAVs en mode droit n'excède pas le quatrième seuil. Les cinquièmes moyens sont mis en oeuvre si les quatrièmes moyens déterminent que la variation du paramètre EA caractéristique pour différents DAVs en mode droit excède le quatrième seuil, et les sixièmes moyens sont aptes à déterminer la présence d'un phénomène de stimulation anodique si les cinquièmes moyens déterminent que la variation du paramètre EA caractéristique pour différents DVVs avec stimulation du ventricule gauche d'abord, n'excède pas le cinquième seuil.

De préférence, le dispositif comprend en outre des moyens de mise à jour de l'énergie de stimulation du ventricule gauche ou droit, mis en oeuvre en réponse à la détermination de la survenue d'une perte de capture respectivement à gauche ou à droite par les sixièmes moyens, et/ou des moyens de mise à jour de l'énergie de stimulation du ventricule gauche ou de modification de la configuration des électrodes de stimulation, mis en oeuvre en réponse à la détermination de la présence d'un phénomène de stimulation anodique par les sixièmes moyens.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est une vue d'ensemble montrant l'implantation de sondes de stimulation des ventricules droit et gauche, avec les différents vecteurs de stimulation susceptibles d'être utilisés dans le cadre d'une stimulation biventriculaire.

La Figure 2 est un schéma par blocs illustrant les différentes étapes de la technique d'analyse et d'optimisation de la stimulation biventriculaire selon l'invention.

La Figure 3 illustre une famille de caractéristiques de variation de l'amplitude du pic EA en fonction du DAV, pour différentes valeurs de DVV.

La Figure 4 illustre deux caractéristiques de variation de l'amplitude du pic EA en fonction du DAV, comparées pour déterminer si elles sont équivalentes ou non en fonction de leur variabilité propre.

La Figure 5 illustre un exemple de résultats de mesure de l'amplitude du PEA en fonction du DAV pour différentes valeurs de DVV, notamment en présence d'un phénomène de stimulation anodique.

La Figure 6 est une illustration sous forme d'histogramme des résultats de la Figure 5, donnant pour différents DVV la moyenne des valeurs de PEA classées en fonction des DVV respectifs.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

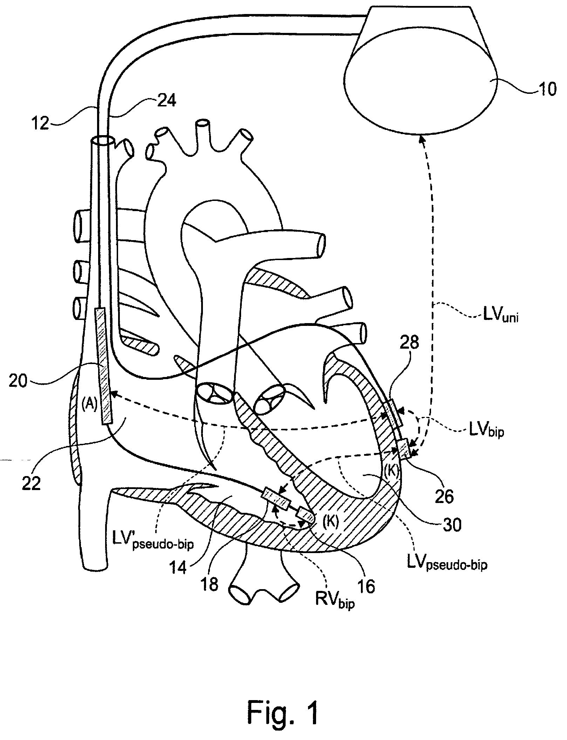

Le procédé de l'invention est mis en oeuvre par des moyens principalement logiciels, au moyen d'algorithmes appropriés, exécutés automatiquement et de façon récurrente par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel. La Figure 1 illustre un dispositif typique de stimulation biventriculaire, où un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18. Cette sonde peut être également pourvue d'un bobinage (*coil*) 20 formant électrode de défibrillation, et permettant également de recueillir un signal endocavitaire (signal EGM) dans l'oreillette droite 22. Pour permettre une stimulation biventriculaire, notamment pour rétablir la synchronisation entre les deux ventricules, le dispositif comporte une deuxième sonde 24, par exemple une sonde disposée dans le réseau coronaire et comportant une ou plusieurs électrodes telles que 26 (électrode distale *tip*) et 28 (électrode proximale *ring*) disposées au voisinage du ventricule gauche 30. Il est ainsi possible d'assurer la stimulation concomitante, ou avec décalage temporel contrôlé (délai interventriculaire DVV) des deux ventricules droit et gauche pour rétablir la synchronisation entre ces deux cavités et améliorer l'hémodynamique générale du patient.

Le ventricule droit (ou RV) est généralement stimulé par application d'impulsions entre les électrodes d'extrémité 16 et 18 de la sonde 12 : configuration de stimulation du ventricule droit désignée "RV$_{bip}$".

En ce qui concerne le ventricule gauche (ou LV), celui-ci peut être stimulé de différentes manières, chaque configuration étant sélectionnée par une commutation appropriée des électrodes au sein du générateur 10. Parmi les configurations possibles du ventricule gauche, on peut ainsi trouver :

- une configuration unipolaire LV$_{uni}$, entre l'une ou l'autre des électrodes 26 ou 28, d'une part, et le boitier (*can*) du générateur 10 ;
- une configuration bipolaire LV$_{bip}$, entre les deux électrodes d'extrémité 26 et 28 de la sonde 24 ;
- une première configuration "pseudo-bipolaire" LV$_{pseudo-bip}$ entre, d'une part, l'une des électrodes d'extrémité de la sonde gauche 24, par exemple l'électrode distale 26 et, d'autre part, l'une des électrodes d'extrémité de la sonde ventriculaire droite 12, par exemple l'électrode proximale droite 18 ;
- une seconde configuration pseudo-bipolaire LV'$_{pseudo-bip}$, entre, d'une part, l'une des électrodes d'extrémité de la sonde gauche 24, par exemple l'électrode proximale 28 et, d'autre part, le bobinage 20 de la sonde droite 12.

Les impulsions électriques sont appliquées par le générateur entre les différentes paires d'électrodes que l'on vient de décrire, de manière à induire localement une dépolarisation sur l'électrode formant cathode, qui est l'électrode distale 16 ou 26 dans le cas d'une stimulation unipolaire ou bipolaire, l'anode étant selon le cas le boitier métallique du générateur 10 ou l'électrode proximale 18 ou 28.

Dans le cas d'une stimulation pseudo-bipolaire du ventricule gauche, la cathode est l'électrode 26 ou 28 placée contre le ventricule gauche, l'anode étant l'électrode 18 ou 20 de la sonde droite, respectivement, utilisée seulement pour définir un retour de courant vers le générateur. Comme on l'a exposé plus haut, dans certaines situations, l'onde de dépolarisation du ventricule gauche ne se crée pas uniquement à la cathode (donc au niveau du ventricule gauche), mais aussi à l'anode (donc au niveau du ventricule droit) : en d'autres termes, l'impulsion destinée à contracter le ventricule gauche va générer également une dépolarisation à droite (phénomène de "stimulation anodique").

Le phénomène de stimulation anodique a pour conséquence que l'onde de dépolarisation correspondant à l'impulsion appliquée à gauche va produire au même moment une dépolarisation dans le ventricule droit, faisant perdre le bénéfice d'une stimulation gauche anticipée pendant la thérapie de resynchronisation.

Le risque d'apparition d'une stimulation anodique est relativement important lors d'un test de seuil de capture, qui commence avec une énergie de stimulation élevée. Ce risque s'amenuise ensuite au fur et à mesure de la réduction de l'amplitude de stimulation, mais l'algorithme de test aura été leurré en interprétant - à tort - la disparition de la stimulation anodique (qu'il n'a pas identifiée comme telle) par une perte de capture, conduisant à une classification erronée des cycles successifs du test entre cycles capturants et non-capturants.

La technique de l'invention remédie à cette difficulté, par une nouvelle technique d'analyse de l'efficacité de la stimulation biventriculaire permettant d'identifier non seulement une perte de capture à droite et/ou à gauche, mais encore et surtout l'apparition d'un phénomène de stimulation anodique.

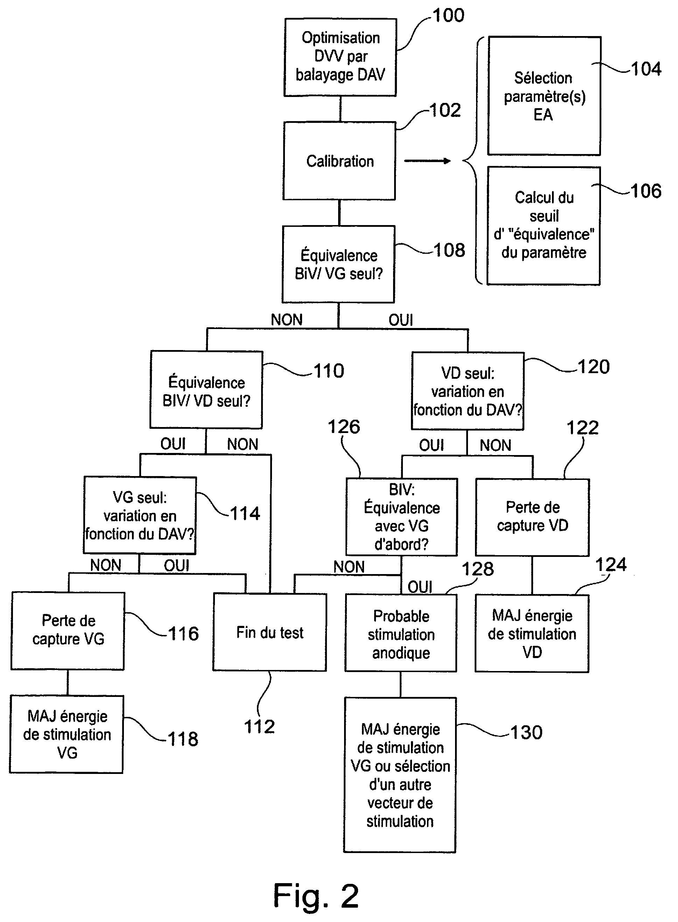

La Figure 2 est un schéma par blocs illustrant les différentes étapes de cette technique d'analyse et d'optimisation de la stimulation biventriculaire selon l'invention.

La première étape (bloc 100) est l'exécution d'un test conventionnel d'optimisation des DAV et DVV par balayage du DAV pour un DVV donné, puis analyse des variations du signal EA résultant de ces modifications du DAV.

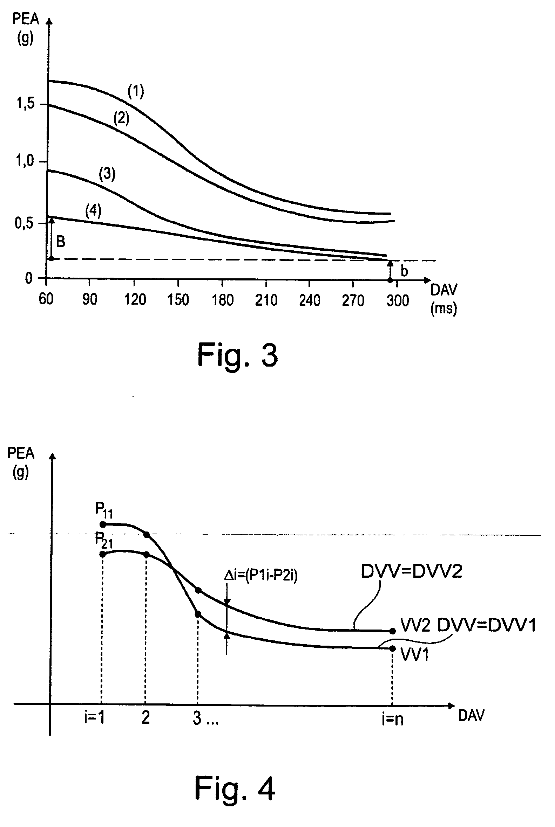

Cette technique est exposée notamment dans le EP 1 736 203 A1 précité, auquel on pourra se référer pour de plus amples détails. Le principe en est illustré sur la Figure 3, qui présente une caractéristique donnant la variation de l'amplitude du premier pic d'accélération endocardiaque (PEA) en fonction du DAV, pour différentes valeurs possibles de DVV (valeurs correspondant aux différentes courbes (1) à (4) de la famille de courbes illustrée).

En cas de stimulation CRT correcte, la caractéristique prend une forme sigmoïde caractéristique, où le DAV varie entre ses deux extrema. Ceci peut être interprété par le fait que la diminution de l'amplitude du PEA avec l'augmentation du DAV est déterminée principalement par :

- la "réserve de contractilité" du myocarde, correspondant au niveau de la ligne de base b (la valeur inférieure limite de PEA pour les DAV les plus longs) ; et
- le "bruit" produit par les valves cardiaques, principalement la valve mitrale, qui détermine l'élévation du niveau d'amplitude au-dessus de cette ligne de base pour les DAV les plus courts (*B* sur la Figure 3).

Ce balayage est effectué pour au moins trois DVV différents, à chaque fois pour au moins six valeurs de DAV, choisies plus courtes que celles de la conduction AV spontanée (intervalle PR/AR) du patient.

L'étape suivante (bloc 102) est une étape propre à l'invention, dite de "calibration", qui a pour objet :

- de déterminer au moins un paramètre EA susceptible de refléter de façon appropriée les différences entre plusieurs configurations de stimulation testées (bloc 104), et
- de déterminer pour le(s) paramètre(s) EA ainsi sélectionné(s) un seuil d' "équivalence" (bloc 106), fonction de la variation intrinsèque du paramètre EA, qui permettra de décider si deux modes de stimulation peuvent être considérés comme produisant substantiellement les mêmes effets (ils seront alors qualifiés d' "équivalents"), ou non.

Les EP 2 495 013 A1 (Sorin CRM) et EP 2 092 885 A1 (ELA Medical) illustrent la manière d'analyser un signal EA recueilli par un capteur d'accélération endocardiaque, et d'en dériver un certain nombre de paramètres représentatifs, ci-après désignés "paramètres EA", parmi lesquels on peut citer :

- la valeur de l'amplitude crête-à-crête de la composante EA1 (ci-après simplement désignée "PEA") ;
- la durée d'apparition du début de la composante EA1, représentée par l'intervalle de temps séparant i) un marqueur temporel de début de cycle cardiaque et ii) le franchissement d'un seuil d'enveloppe d'énergie de la composante EA1 ;
- l'intervalle de temps séparant i) le franchissement du seuil d'enveloppe d'énergie de la composante EA1 de ii) l'instant du pic de l'enveloppe d'énergie de la composante EA1 ;
- la durée de la systole, représentée par l'intervalle de temps séparant le début de la composante EA1 du début de la composante EA2 ; et/ou
- des paramètres EA composites combinant les précédents.

On pourra se référer aux deux documents précités pour plus de détails sur la manière dont sont déterminés ces paramètres EA, ainsi que d'autres caractéristiques des composantes EA1 et EA2.

L'objet de l'étape 104 est de sélectionner, parmi les divers paramètres EA possibles, un paramètre EA (ou éventuellement, plusieurs de ces paramètres EA) permettant d'évaluer au mieux l'effet d'une modification de la configuration et du mode de stimulation, dans le but de détecter la présence d'une éventuelle stimulation anodique et/ou d'une perte de capture ventriculaire gauche ou droite. Ce paramètre EA est choisi par un opérateur.

Les différents modes de stimulation possibles qui seront testés sur la base du paramètre EA ainsi sélectionné sont :

- stimulation du seul ventricule gauche (LV), ci-après "mode G" ou "VG seul" ;

- stimulation du seul ventricule droit (RV), ci-après "mode D" ou "VD seul" ;
- stimulation concomitante des deux ventricules, ci-après "mode biventriculaire" ou "BiV", avec application d'un DVV négatif nul ou positif.

L'étape 106 de calcul du "seuil d'équivalence" du paramètre EA sélectionné a pour objet de définir un seuil permettant de distinguer entre deux situations hémodynamiques différentes, de façon décorrélée de la variabilité intrinsèque du paramètre EA.

Si l'on prend comme exemple de paramètre EA le PEA, c'est-à-dire la valeur moyenne de l'amplitude crête-à-crête du premier pic EA pour les différents DAV appliqués, on peut par exemple définir un indicateur d'équivalence par le pourcentage de l'erreur quadratique moyenne (RMSE) entre deux conditions de stimulation avec des DVV différents, DVV1 et DVV2 :

$$\text{RMSE} = 100 \times \sqrt{\frac{\sum_{i=1}^{n}\left(\frac{\Delta_i}{P1i}\right)^2}{n}}$$

n étant le nombre de DAV testés pour chaque condition de stimulation (pour DVV1 et pour DVV2), et

$$\Delta_i = (P1i - P2i),$$

$P1i$ et $P2i$ étant les valeurs du paramètre EA considéré (PEA en l'espèce) mesurées pour le $i^{\text{ème}}$ DAV, respectivement pour DVV = DVV1 et pour DVV = DVV2.

La Figure 4 illustre un exemple de variation de ces paramètres, et l'on pourra décider que si, par exemple, la valeur de RMSE entre DVV1 et DVV2 est inférieure à un seuil prédéfini (par exemple 10 %, mais cette valeur peut aussi dépendre de la variabilité intrinsèque du paramètre EA sur des cycles consécutifs en gardant un même DVV), alors les deux configurations de stimulation seront considérées comme "équivalentes".

Cet indicateur d'équivalence sera également utilisé pour évaluer cette même "équivalence", entre différents modes de stimulation (VG seul, VD seul ou BiV).

Une fois la phase de calibration (bloc 102) achevée, l'évaluation de l'efficacité de la stimulation biventriculaire commence par un test d'équivalence entre une stimulation en mode BiV et une stimulation en mode VG seul (bloc 108).

Une réponse négative à ce test correspond à un cas normal, dans la mesure où la stimulation biventriculaire révèle une situation hémodynamique différente (donc non équivalente) d'une stimulation du seul ventricule gauche. L'algorithme teste alors l'équivalence entre les modes BiV et VD seul (bloc 110).

Une réponse négative correspond ici encore au cas typique où la stimulation biventriculaire est normalement plus efficace que la stimulation en mode VD seul. L'analyse prend alors fin en retournant un résultat indiquant que la stimulation biventriculaire est conforme et efficace (bloc 112). Si en revanche une équivalence est trouvée entre les modes de stimulation BiV et VD seul (bloc 110), ceci signifie que la stimulation biventriculaire n'a rien apporté par rapport à une stimulation du seul ventricule droit. L'algorithme analyse alors (bloc 114), en mode VG seul, la variation du paramètre EA en fonction du DAV.

Si la variation du DAV ne produit aucun effet, ceci signifie qu'il y a une perte de capture sur le ventricule gauche (bloc 116) et qu'il y a donc lieu de déclencher un test de capture pour réévaluer l'énergie de stimulation du ventricule gauche et l'ajuster à un niveau plus élevé (bloc 118).

Si en revanche une variation du DAV induit une variation effective du paramètre EA, la suspicion de perte de capture n'a plus lieu d'être et la stimulation doit être reconnue conforme (bloc 112).

Si, lors de la comparaison entre les modes de stimulation BiV et VG seul (bloc 108) ces deux modes avaient été reconnus comme équivalents, cette situation est anormale car elle signifie que sur le plan hémodynamique la stimulation biventriculaire n'a rien apporté par rapport à une stimulation du seul ventricule gauche. Cette anomalie peut provenir d'une perte de capture à droite ou bien d'une stimulation anodique, causes qu'il convient de discriminer car les remèdes à apporter ne seront pas les mêmes.

Cette situation de probable stimulation anodique (bloc 128) se présente dans le contexte suivant :

- programmation d'un vecteur de stimulation pseudo-bipolaire ($LV_{pseudo-bip}$ ou $LV'_{pseudo-bip}$ sur la Figure 1) ;
- présence d'une variabilité du paramètre EA pour des DAV différents en mode VD seul (test du bloc 120) ; et
- équivalence des valeurs du paramètre EA entre une stimulation biventriculaire pure (DVV = 0) et une ou plusieurs configurations de stimulation de type LR, c'est-à-dire avec une stimulation d'abord à gauche (test du bloc 126).

L'algorithme teste alors l'effet d'une variation du DAV sur le paramètre EA, en mode de stimulation VD seul (bloc 120, semblable au bloc 114 mais pour une stimulation à droite).

L'absence de variation suite à ce test révèle une perte de capture à droite (bloc 122), à laquelle il convient de remédier par déclenchement d'un test de capture et mise à jour de l'énergie de stimulation du ventricule droit (bloc 124).

Si en revanche une modification du DAV induit effectivement une variation du paramètre EA (bloc 120), la présence de cette variabilité laisse supposer l'apparition d'un phénomène de stimulation anodique.

Pour lever ce doute, le test se poursuit en évaluant (bloc 126) l'équivalence entre configurations avec des DVV différents, mais où une stimulation côté gauche précède la stimulation côté droit (configuration "gauche d'abord" ou "LR") :

- si une équivalence est trouvée entre ces configurations, ceci signifie que sur le plan hémodynamique la variation du DVV ne produit aucun résultat entre la première stimulation à gauche et la seconde à droite, situation typique de la présence d'une stimulation anodique (bloc 128). Il convient alors de mettre à jour l'énergie de stimulation du ventricule gauche, ou de modifier la polarité de la stimulation de ce ventricule gauche par un choix d'électrodes différent, de manière à définir un autre vecteur de stimulation (bloc 130) ;
- si en revanche la modification du DVV en configuration "LR" induit des modifications du paramètre EA (non-équivalence), il n'y a pas de stimulation anodique (bloc 112) et une modification de l'énergie de stimulation ou du vecteur de stimulation ne sera pas requise.

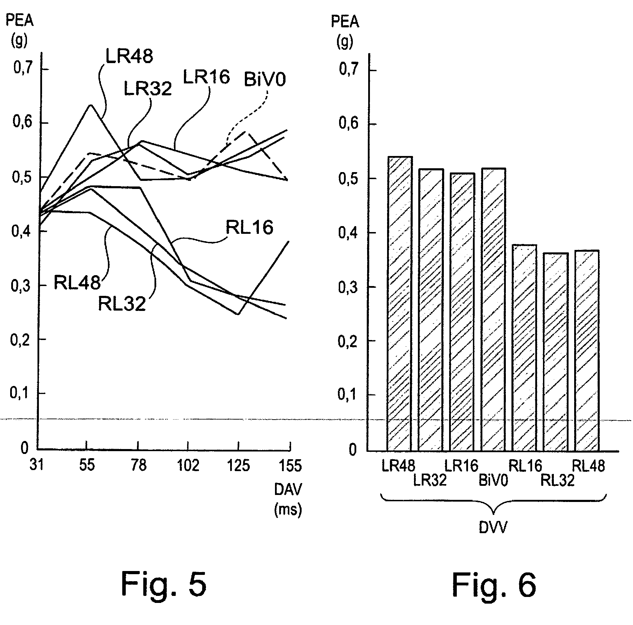

La Figure 5 illustre un exemple de résultats de mesure de l'amplitude du PEA en fonction du DAV pour différentes valeurs de DVV, notamment en présence d'un phénomène de stimulation anodique, ces résultats étant présentés sous forme d'histogramme sur la Figure 6, avec pour différents DVV la moyenne des valeurs de PEA classées en fonction des DVV respectifs.

Sur ces figures les indications "LR*nn*" correspondent à une stimulation du ventricule gauche d'abord, suivie de celle du ventricule droit, la valeur numérique *nn* indiquant le DVV appliqué (par exemple 16, 32 et 48 ms). Les indications "RL*nn*" correspondent à la situation inverse, où le ventricule droit est stimulé le premier. La référence "BiV0" indique une stimulation biventriculaire à DVV nul, c'est-à-dire appliquée simultanément sur les deux ventricules.

L'exemple illustré correspond à un cas clinique dans lequel une stimulation anodique est présente lors de la stimulation du ventricule gauche. Comme on peut le voir sur l'histogramme de la Figure 6, les valeurs du paramètre EA associées aux configurations avec une stimulation du ventricule gauche en premier (configurations LR) sont toutes équivalentes à celles mesurées en configuration biventriculaire pure BiV0, révélant l'inefficacité de l'application d'un DVV, quelle que soit la valeur de celui-ci. En revanche, pour une stimulation du ventricule droit en premier (dans l'exemple, configurations RL16, RL32 et RL48) une différence significative apparait par rapport à une stimulation biventriculaire pure BiV0, révélant l'efficacité de l'application d'un DVV. On observe d'ailleurs sur les caractéristiques PEA/DAV de la Figure 5 que les caractéristiques RL16, RL32 et RL48 sont très différentes des configurations LR16, LR32 et LR48.

**Revendications**

1.  Un dispositif médical implantable actif de resynchronisation cardiaque par stimulation biventriculaire, comportant :

    - des moyens (10-28) de détection d'événements auriculaires et ventriculaires ;
    - des moyens (10-28) de stimulation des ventricules droit et gauche ;
    - des moyens aptes à appliquer aux moyens de stimulation un délai atrioventriculaire, DAV, compté à partir de la détection d'un évènement auriculaire spontané ou stimulé et à l'issue duquel une stimulation du ventricule droit est appliquée en l'absence d'évènement ventriculaire spontané détecté ;
    - des moyens aptes à appliquer aux moyens de stimulation un délai interventriculaire, DVV, entre les instants respectifs de stimulation des ventricules droit et gauche ; et
    - des moyens de contrôle des moyens de stimulation, aptes à opérer sélectivement soit en un mode droit (VD seul) où seul le ventricule droit est stimulé, soit en un mode gauche (VG seul) où seul le ventricule gauche est stimulé, soit en un mode biventriculaire (BiV) où les deux ventricules sont stimulés conjointement avec ou sans application d'un DVV,

    **caractérisé en ce qu'**il comprend en outre :

    - un capteur apte à délivrer un signal d'accélération endocardiaque, EA ;
    - des moyens aptes à extraire du signal EA au moins un paramètre EA caractéristique prédéterminé ; et
    - des moyens d'évaluation de la stimulation biventriculaire, comprenant :

        · des premiers moyens (108), aptes à comparer les valeurs du paramètre EA caractéristique obtenues respectivement en mode biventriculaire et en mode gauche et à déterminer si l'écart entre ces valeurs excède un premier seuil prédéterminé ;
        · des deuxièmes moyens (110), aptes à comparer les valeurs du paramètre EA caractéristique obtenues respectivement en mode biventriculaire et en mode droit et à déterminer si l'écart entre ces valeurs excède un deuxième seuil prédéterminé ;
        · des troisièmes moyens (114), aptes à déterminer si la variation du paramètre EA caractéristique pour différents DAVs en mode gauche excède un troisième seuil donné ;
        · des quatrièmes moyens (120), aptes à déterminer si la variation du paramètre EA caractéristique pour différents DAVs en mode droit excède un quatrième seuil donné ;
        · des cinquièmes moyens (126), aptes à comparer les valeurs du paramètre EA caractéristique obtenues en mode biventriculaire respectivement avec un DVV négatif ou positif et à déterminer si l'écart entre ces valeurs excède un cinquième seuil prédéterminé ; et
        · des sixièmes moyens, aptes à déterminer en réponse aux premiers, deuxièmes, troisièmes, quatrièmes et cinquièmes moyens, la survenue d'une éventuelle perte de capture à gauche (116) ou à droite (122), ou la présence d'un phénomène de stimulation anodique (128).

2.  Le dispositif de la revendication 1, comprenant en outre :

    - des moyens (100) de test préalable d'une pluralité de configurations de stimulation avec différents DVVs pour une pluralité de DAVs ; et
    - des moyens (104) de sélection préalable du paramètre EA caractéristique prédéterminé parmi plusieurs paramètres EA possibles, en fonction des résultats produits par les moyens de test préalable d'une pluralité de configurations.

3.  Le dispositif de la revendication 1, comprenant en outre :

    - des moyens (100) de test préalable d'une pluralité de configurations de stimulation avec différents DVVs pour une pluralité de DAVs ; et
    - des moyens (106) de détermination du premier seuil, en fonction des résultats produits par les moyens de test préalable d'une pluralité de configurations.

4.  Le dispositif de la revendication 1, dans lequel :

    - les deuxièmes moyens (110) comprennent des moyens de commande conditionnelle en réponse à la détermination par les premiers moyens (108) que l'écart entre les valeurs du paramètre EA caractéristique obtenues en mode biventriculaire et en mode gauche excède le premier seuil ;
    - les troisièmes moyens (114) comprennent des moyens de commande conditionnelle en réponse à la détermination par les deuxièmes moyens (110) que l'écart entre les valeurs du paramètre EA caractéristique obtenues en mode biventriculaire et en mode droit n'excède pas le deuxième seuil ; et

- les sixièmes moyens sont aptes à déterminer la survenue d'une perte de capture à gauche (116) en réponse à la détermination par les troisièmes moyens (114) que la variation du paramètre EA caractéristique pour différents DAVs en mode gauche n'excède pas le troisième seuil.

5. Le dispositif de la revendication 1, dans lequel :

- les quatrièmes moyens (120) comprennent des moyens de commande conditionnelle en réponse à la détermination par les premiers moyens (108) que l'écart entre les valeurs du paramètre EA caractéristique obtenues en mode biventriculaire et en mode gauche n'excède pas le premier seuil ; et
- les sixièmes moyens sont aptes à déterminer la survenue d'une perte de capture à droite (122) en réponse à la détermination par les quatrièmes moyens (120) que la variation du paramètre EA caractéristique pour différents DAVs en mode droit n'excède pas le quatrième seuil.

6. Le dispositif de la revendication 1, dans lequel :

- les quatrièmes moyens (120) comprennent des moyens de commande conditionnelle en réponse à la détermination par les premiers moyens (108) que l'écart entre les valeurs du paramètre EA caractéristique obtenues en mode biventriculaire et en mode gauche n'excède pas le premier seuil ;
- les cinquièmes moyens (126) comprennent des moyens de commande conditionnelle en réponse à la détermination par les quatrièmes moyens (120) que la variation du paramètre EA caractéristique pour différents DAVs en mode droit excède le quatrième seuil ; et
- les sixièmes moyens sont aptes à déterminer la présence d'un phénomène de stimulation anodique (128) en réponse à la détermination par les cinquièmes moyens (126) que la variation du paramètre EA caractéristique pour différents DVVs avec stimulation du ventricule gauche d'abord, n'excède pas le cinquième seuil.

7. Le dispositif de la revendication 1, comprenant en outre :

- des moyens (118, 124) de mise à jour de l'énergie de stimulation du ventricule gauche ou droit, en réponse à la détermination de la survenue d'une perte de capture respectivement à gauche (116) ou à droite (122) par les sixièmes moyens.

8. Le dispositif de la revendication 1, comprenant en outre :

- des moyens (130) de mise à jour de l'énergie de stimulation du ventricule gauche ou de modification de la configuration des électrodes de stimulation, en réponse à la détermination de la présence d'un phénomène de stimulation anodique (128) par les sixièmes moyens.

**Patentansprüche**

1. Aktive implantierbare medizinische Vorrichtung zur kardialen Resynchronisation durch biventrikuläre Stimulation, umfassend:

- Mittel (10-28) zum Detektieren von atrialen und ventrikulären Ereignissen;
- Mittel (10-28) zur Stimulation des rechten und des linken Ventrikels;
- Mittel, die geeignet sind, auf die Mittel zur Stimulation eine atrioventrikuläre Verzögerung DAV anzuwenden, die ab der Detektion eines spontanen oder stimulierten atrialen Ereignisses beginnt, und nach der in Abwesenheit eines detektierten spontanen ventrikulären Ereignisses eine Stimulation des rechten Ventrikels angewendet wird;
- Mittel, die geeignet sind, auf die Mittel zur Stimulation eine interventrikuläre Verzögerung DVV zwischen den jeweiligen Zeitpunkten einer Stimulation des rechten und des linken Ventrikels anzuwenden; und
- Kontrollmittel der Mittel zur Stimulation, die geeignet sind, selektiv entweder in einem rechten Modus (nur VD), wo nur das rechte Ventrikel stimuliert wird, oder in einem linken Modus (nur VG), wo nur das linke Ventrikel stimuliert wird, oder in einem biventrikulären Modus (BiV), wo die zwei Ventrikel mit oder ohne Anwenden einer DVV gemeinsam stimuliert werden, zu arbeiten,

**dadurch gekennzeichnet, dass** sie ferner Folgendes aufweist:

- einen Sensor, der geeignet ist, ein endokardiales Beschleunigungssignal EA auszugeben;
- Mittel, die geeignet sind, aus dem Signal EA mindestens einen vorbestimmten, charakteristischen Parameter EA zu extrahieren; und
- Mittel zum Bewerten der biventrikulären Stimulation, umfassend:

  · erste Mittel (108), die geeignet sind, die Werte des charakteristischen Parameters EA, die jeweils im biventrikulären Modus und im linken Modus erhalten werden, zu vergleichen und zu bestimmen, ob die Differenz zwischen diesen Werten einen vorbestimmten ersten Schwellenwert übersteigt;

· zweite Mittel (110), die geeignet sind, die Werte des charakteristischen Parameters EA, die jeweils im biventrikulären Modus und im rechten Modus erhalten werden, zu vergleichen und zu bestimmen, ob die Differenz zwischen diesen Werten einen vorbestimmten zweiten Schwellenwert übersteigt;

· dritte Mittel (114), die geeignet sind zu bestimmen, ob die Variation des charakteristischen Parameters EA für verschiedene DAVs im linken Modus einen gegebenen dritten Schwellenwert übersteigt;

· vierte Mittel (120), die geeignet sind zu bestimmen, ob die Variation des charakteristischen Parameters EA für verschiedene DAVs im rechten Modus einen gegebenen vierten Schwellenwert übersteigt;

· fünfte Mittel (126), die geeignet sind, die Werte des charakteristischen Parameters EA, die im biventrikulären Modus jeweils mit einer negativen und einer positiven DVV erhalten werden, zu vergleichen und zu bestimmen, ob die Differenz zwischen diesen Werten einen vorbestimmten fünften Schwellenwert übersteigt; und

· sechste Mittel, die geeignet sind, als Antwort auf die ersten, zweiten, dritten, vierten und fünften Mittel das Auftreten eines eventuellen Verlustes des Einfangs links (116) oder rechts (122) oder die Anwesenheit eines Phänomens der anodischen Stimulation (128) zu bestimmen.

2. Vorrichtung von Anspruch 1, ferner umfassend:

- Mittel (100) zum vorherigen Prüfen mehrerer Stimulationskonfigurationen mit verschiedenen DVVs für mehrere DAVs; und
- Mittel (104) zum vorherigen Auswählen des vorbestimmten, charakteristischen Parameters EA aus mehreren möglichen Parametern EA in Abhängigkeit der Ergebnisse, die durch die Mittel zum vorherigen Prüfen mehrerer Konfigurationen produziert werden.

3. Vorrichtung von Anspruch 1, ferner umfassend:

- Mittel (100) zum vorherigen Prüfen mehrerer Stimulationskonfigurationen mit verschiedenen DVVs für mehrere DAVs; und
- Mittel (106) zum Bestimmen eines ersten Schwellenwerts in Abhängigkeit der Ergebnisse, die durch die Mittel zum vorherigen Prüfen mehrerer Konfigurationen produziert werden.

4. Vorrichtung von Anspruch 1, wobei:

- die zweiten Mittel (110) Mittel zum bedingten Steuern als Antwort auf das Bestimmen durch die ersten Mittel (108), dass die Differenz zwischen den Werten des charakteristischen Parameters EA, die im biventrikulären Modus und im linken Modus erhalten werden, den ersten Schwellenwert übersteigt, aufweisen;
- die dritten Mittel (114) Mittel zum bedingten Steuern als Antwort auf das Bestimmen durch die zweiten Mittel (110), dass die Differenz zwischen den Werten des charakteristischen Parameters EA, die im biventrikulären Modus und im rechten Modus erhalten werden, den zweiten Schwellenwert nicht übersteigt, aufweisen; und
- die sechsten Mittel geeignet sind, das Auftreten eines Verlustes des Einfangs links (116) als Antwort auf das Bestimmen durch die dritten Mittel (114), dass die Variation des charakteristischen Parameters EA für verschiedene DAVs im linken Modus den dritten Schwellenwert nicht übersteigt, zu bestimmen.

5. Vorrichtung von Anspruch 1, wobei:

- die vierten Mittel (120) Mittel zum bedingten Steuern als Antwort auf das Bestimmen durch die ersten Mittel (108), dass die Differenz zwischen den Werten des charakteristischen Parameters EA, die im biventrikulären Modus und im linken Modus erhalten werden, den ersten Schwellenwert nicht übersteigt, aufweisen; und
- die sechsten Mittel geeignet sind, das Auftreten eines Verlustes des Einfangs rechts (122) als Antwort auf das Bestimmen durch die vierten Mittel (120), dass die Variation des charakteristischen Parameters EA für verschiedene DAVs im rechten Modus den vierten Schwellenwert nicht übersteigt, zu bestimmen.

6. Vorrichtung von Anspruch 1, wobei:

- die vierten Mittel (120) Mittel zum bedingten Steuern als Antwort auf das Bestimmen durch die ersten Mittel (108), dass die Differenz zwischen den Werten des charakteristischen Parameters EA, die im biventrikulären Modus und im linken Modus erhalten werden, den ersten Schwellenwert nicht übersteigt, aufweisen; und
- die fünften Mittel (126) Mittel zum bedingten Steuern als Antwort auf das Bestimmen durch die vierten Mittel (120), dass die Variation des charakteristischen Parameters EA für verschiedene DAVs im rechten Modus den vierten Schwellenwert übersteigt, aufweisen; und
- die sechsten Mittel geeignet sind, die Anwesenheit eines Phänomens der anodischen Stimulation (128) als Antwort auf das Bestimmen durch die fünften Mittel (126), dass die Variation des charakteristischen Parameters EA für verschiedene DVVs zuerst mit Stimulation des linken Ventrikels den fünften Schwellenwert nicht übersteigt, zu bestimmen.

7. Vorrichtung von Anspruch 1, ferner umfassend:

- Mittel (118, 124) zum Aktualisieren der Energie zur Stimulation des linken oder rechten Ventrikels als Antwort auf das Bestimmen des Auftretens eines Verlustes des Einfangs jeweils links (116) oder rechts (122) durch die sechsten Mittel.

8. Vorrichtung von Anspruch 1, ferner umfassend:

- Mittel (130) zum Aktualisieren der Energie zur Stimulation des linken Ventrikels oder zum Modifizieren der Konfiguration der Stimulationselektroden als Antwort auf das Bestimmen der Anwesenheit eines Phänomens der anodischen Stimulation (128) durch die sechsten Mittel.

**Claims**

1. An active implantable medical device for cardiac resynchronization by biventricular stimulation, including:

- means (10-28) for detecting atrial and ventricular events;
- means (10-28) for stimulating the right and left ventricles;
- means adapted to apply to the stimulation means an atrioventricular delay, DAV, counted from the detection of a spontaneous or stimulated atrial event and at the end of which a stimulation of the right ventricle is applied in the absence of a spontaneous ventricular event detected;
- means adapted to apply to the stimulation means an interventricular delay, DVV, between the respective instants of stimulation of the right and left ventricles; and
- means for controlling the stimulation means, adapted to operate selectively either in right mode (VD alone) where only the right ventricle is stimulated, or in left mode (VG alone) where only the left ventricle is stimulated, or in biventricular mode (BiV) where the two ventricles are jointly stimulated with application or not of a DVV,

**characterized in that** it further comprises:

- a sensor adapted to deliver an endocardial acceleration signal, EA;
- means adapted to extract from the signal EA at least one predetermined characteristic EA parameter; and
- means for evaluating the biventricular stimulation, comprising:

· first means (108), adapted to compare the values of the characteristic EA parameter obtained in biventricular mode and left mode, respectively, and to determine if the difference between these values exceeds a first predetermined threshold;
· second means (110), adapted to compare the values of the characteristic EA parameter obtained in biventricular mode and right mode, respectively, and to determine if the difference between these values exceeds a second predetermined threshold;
· third means (114), adapted to determine if the variation of the characteristic EA parameter for different DAV in left mode exceeds a third given threshold;
· fourth means (120), adapted to determine if the variation of the characteristic EA parameter for different DAV in right mode exceeds a fourth given threshold;
· fifth means (126), adapted to compare the values of the characteristic EA parameter obtained in biventricular mode with a negative or a positive DVV, respectively, and to determine if the difference between these values exceeds a fifth predetermined threshold; and
· sixth means, adapted to determine, in response to the first, second, third, fourth and fifth means, the occurrence of a potential loss of capture on the left side (116) or the right side (122), or the presence of a phenomenon of anodic stimulation (128).

2. The device of claim 1, further comprising:

- means (100) for previously testing a plurality of stimulation configurations with different DDV for a plurality of DAV; and
- means (104) for previously selecting the predetermined characteristic EA parameter among several possible EA parameters, as a function of the results produced by the means for previously testing a plurality of configurations.

3. The device of claim 1, further comprising:

- means (100) for previously testing a plurality of stimulation configurations with different DDV for a plurality of DAV; and

- means (106) for determining the first threshold, as a function of the results produced by the means for previously testing a plurality of configurations.

4. The device of claim 1, wherein:

- the second means (110) comprise means for conditional control in response to the determination by the first means (108) that the difference between the values of the characteristic EA parameter obtained in biventricular mode and in left mode exceeds the first threshold;
- the third means (114) comprise means for conditional control in response to the determination by the second means (110) that the difference between the values of the characteristic EA parameter obtained in biventricular mode and in right mode does not exceed the second threshold; and
- the sixth means are adapted to determine the occurrence of a loss of capture on the left side (116) in response to the determination by the third means (114) that the variation of the characteristic EA parameter for different DAV in left mode does not exceed the third threshold.

5. The device of claim 1, wherein:

- the fourth means (120) comprise means for conditional control in response to the determination by the first means (108) that the difference between the values of the characteristic EA parameter obtained in biventricular mode and in left mode does not exceed the first threshold; and
- the sixth means are adapted to determine the occurrence of a loss of capture on the right side (122) in response to the determination by the fourth means (120) that the variation of the characteristic EA parameter for different DAV in right mode does not exceed the fourth threshold.

6. The device of claim 1, wherein:

- the fourth means (120) comprise means for conditional control in response to the determination by the first means (108) that the difference between the values of the characteristic EA parameter obtained in biventricular mode and in left mode does not exceed the first threshold;
- the fifth means (126) comprise means for conditional control in response to the determination by the fourth means (120) that the variation of the characteristic EA parameter for different DAV in right mode exceeds the fourth threshold; and

- the sixth means are adapted to determine the presence of a phenomenon of anodic stimulation (128) in response to the determination by the fifth means (126) that the variation of the characteristic EA parameter for different DVV, with stimulation of the left ventricle first, does not exceed the fifth threshold.

7. The device of claim 1, further comprising:

- means (118, 124) for updating the energy of stimulation of the left or right ventricle, in response to the determination of the occurrence of a loss of capture on the left side (116) or the right side (122), respectively, by the sixth means.

8. The device of claim 1, further comprising:

- means (130) for updating the energy of stimulation of the left ventricle or for modifying the configuration of the stimulation electrodes, in response to the determination of the presence of a phenomenon of anodic stimulation (128) by the sixth means.

Fig. 1

Fig. 2

Fig. 3

Fig. 4

Fig. 5

Fig. 6

**EP 2 803 385 B1**

## RÉFÉRENCES CITÉES DANS LA DESCRIPTION

**Documents brevets cités dans la description**

- EP 1108446 A1
- EP 0515319 A1
- EP 1736203 A1

- US 6687545 B1
- EP 2495013 A1
- EP 2092885 A1